# EUROPEAN PATENT SPECIFICATION

(11) **EP 3 925 974 B1**
(45) Date of publication and mention of the grant of the patent: **01.05.2024**
(21) Application number: 19915275.2
(22) Date of filing: 13.02.2019
(51) Int. Cl.: C07K 14/78, C14B 13/00

(54) **METHOD FOR OBTAINING A MODIFIED COLLAGEN ABSORBENT PRODUCT FROM LEATHER**
VERFAHREN ZUR HERSTELLUNG EINES MODIFIZIERTEN KOLLAGENABSORBIERENDEN PRODUKTES AUS LEDER
PROCÉDÉ POUR OBTENIR UN PRODUIT ABSORBANT DE COLLAGÈNE MODIFIÉ À PARTIR DU CUIR

(43) Date of publication of application: 22.12.2021
(73) Proprietor: REGIONAL DE NEGOCIOS COMERCIALES 2022, S.L., 41320 Cantillana, Sevilla (ES)
(72) Inventor: ITURRIAGA ZAGAL, Francisco Fernando, 03830 MURO DE ALCOY (ALICANTE) (ES)
(74) Representative: Codoñer Molina, Vicente
(86) International application number: PCT/ES2019/070079
(87) International publication number: WO 2020/165467

(56) References cited:
- ES-A1- 467 778
- US-A- 5 731 418
- US-A- 5 731 418

## Description

### Field of the Invention

The present invention relates to the industry dedicated to producing products for absorbing substances such as hydrocarbons, vegetable oils and synthetic oils, and more specifically to the industry dedicated to producing these products from leather scraps.

### State of the Art

Currently, the need to take care of the environment, *inter alia,* through the absorption of harmful spilled substances, is widely known. These substances include hydrocarbons, vegetable oils and synthetic oils.

Said substances may be found to be detrimentally spilled in liquid media, such as, for example, oceans, seas, lakes, marshes and rivers. Likewise, these substances are found to be detrimentally spilled on solid media, such as, for example, soil and cement floors or floors of any other material for said purpose.

In order to absorb said substances, obtaining products from leather scraps is known. In this sense, initially, a method or procedure was used that basically consisted of exposing the leather scraps to the open air for their drying. To do this, at least one person with very simple tools was dedicated to moving around said scraps to standardize the drying of the leather.

This solution has the drawback of being dependent on climatic conditions, which hinders the production of the product. Furthermore, this solution undesirably leads to a long period of time, in terms of months, for it to be performed.

Nowadays, mainly in order to accelerate the procedure, unnatural means are used for the drying of the previously collected leather scraps. Furthermore, using unnatural means, enables that the drying of the leather obtains a result that is not conditioned by climatic or geographical conditions. This solution consists of using unnatural means, such as ovens, so that the leather is exposed to temperatures above 500°C, which considerably accelerates drying.

This solution, nevertheless, leads to the production of poor-quality absorbent products, i.e., of a quality that can be clearly improved.

In light of the described disadvantages or limitations offered by the currently existing solutions, a new solution is necessary that makes it possible to obtain the product for absorbing substances such as those cited in a controlled manner and within acceptable time periods, without sacrificing the quality of the product produced.

Document US5731418A discloses a collagen-based sorbent polymer and a method of preparing the same

### Summary of the invention

In order to fulfil this objective and resolve the technical problems discussed so far, in addition to providing additional advantages that may be derived later, the present invention provides a method, or procedure, for obtaining a modified collagen absorbent product from leather.

The present method comprises the steps of aerating flat leather pieces with a molecular structure during a time period of between 15 and 45 days; collecting the leather pieces with an initial relative humidity value of 45% or less; grading the leather pieces by size; obtaining the leather pieces with a maximum final length of between 0.5 and 1.5 mm, and preferably between 0.75 and 3 mm; placing the leather pieces in a drying oven; applying heat in the drying oven so that the leather pieces are exposed to a temperature of between 120°C and 285°C during a time period;
controlling the temperature applied during the time period so that the relative humidity is reduced to a final value of between 0.5 and 5% in the leather pieces; and reducing the temperature of the leather pieces to an ambient temperature; in addition to the step of having at one's disposal or obtaining the product from leather.

In this sense, the modified collagen product obtained has hollow cells so that it can store or retain all types of hydrocarbons and oils, both vegetable and synthetic. Likewise, the modified collagen product obtained has the capacity of absorbing, by capillarity, all types of hydrocarbons and oils, both vegetable and synthetic, whilst being hydrophobic.

Preferably, the initial relative humidity value is equal to or less than 35%. Additionally or alternatively, the parts are graded by size using mechanical sieving.

In the present method, leather pieces are obtained with a maximum intermediate length of between 0.3 and 3 cm, before being obtained according to the maximum final length. Likewise, between obtaining the leather pieces according to the maximum intermediate length and according to the maximum final length, another grading of the pieces by sizes can be performed preferably by means of another mechanical sieving.

Preferably, the drying oven is made of stainless steel and/or galvanized steel. Additionally or alternatively, preferably, the drying oven has a longitudinal configuration such that the leather pieces are displaced longitudinally along same during the application of the temperature.

Likewise, the drying oven preferably has fins to move around and displace the leather pieces along it during the application of the temperature. Additionally or alternatively, preferably, the drying oven has a particulate filter disposed to collect particles generated by the application of heat to the leather pieces.

In the present method, the temperature in the drying oven can be detected so that a regulation is performed to keep the temperature of the leather pieces within a desired range.

Preferably, the time period of heat application is dependent on the temperature applied and relative humidity of the leather pieces.

According to one option of the present method, before the leather pieces are placed in the drying oven, the leather pieces have the maximum final length.

According to another option of the present method, before the leather pieces with the maximum final length are obtained, the leather pieces are obtained according to the final relative humidity value.

Preferably, the temperature reduction is performed by exposure to ambient temperature.

### Brief description of the figures

Figure 1 shows an enlarged view of localized collagen in a piece of leather before a method for obtaining a modified collagen absorbent product from leather, object of the invention, is applied.
Figure 2 shows an enlarged view of the modified collagen product obtained after the method for obtaining a modified collagen absorbent product from leather, object of the invention, has been applied.

### Detailed description of the invention

The present invention relates to a method for obtaining a modified collagen absorbent product from leather. Preferably, the method involves recycling organic waste derived from a tanning process, wherein collagen from the skin is stabilized by tanning agents. Thus, preferably, the method comprises a collection of leather pieces or shavings with respect to a tannery. Thus, instead of going to a landfill, these leather pieces or shavings are used to obtain the modified collagen product.

The leather pieces are laid out so that they are exposed to natural aeration during a time period. In this sense, the leather pieces are laid out outdoors or exposed to the environment so that the leather pieces are in contact with nature. Optionally, the leather pieces are periodically moved around to provide natural aeration in a uniform manner.

The aeration time period is preferably between 15 and 45 days, more preferably between 20 and 40 days, and, even more preferably between 22 and 33 days. The leather pieces have an initial humidity according to an initial value, which is unknown before the leather pieces are exposed to said natural aeration. Hence, the time period is additionally dependent on an initial relative humidity value, so that the application of natural aeration ends with the initial relative humidity value preferably being equal to or less than 45%, more preferably equal to or less than 40%, even more preferably equal to or less than 35% and, yet even more preferably equal to or less than 30%.

The method comprises a first grading of the pieces by sizes. Thus, the method comprises performing a first mechanical sieving such that the leather pieces are identified and selected according to a first size. This first size is defined so that a maximum intermediate length of between 0.3 and 3 cm, and more preferably between 0.5 and 2 cm, is definable in each of the leather pieces.

To obtain the leather pieces according to the first size, a first reduction in the size of said pieces is totally or partially applied. Preferably, this reduction in the size of the leather pieces is carried out by grinding them. In this way, the reduction, or grinding, is preferably applied only to leather pieces with the maximum intermediate length greater than 3 cm, and more preferably greater than 2 cm. Thus, therefore, a first uniformity is obtained in the size of the leather pieces.

The method comprises a second grading of the pieces by sizes. In this sense, the method comprises performing a second mechanical sieving such that the leather pieces are identified and selected according to a second size. This second size is defined so that a maximum final length of between 0.5 and 5 mm, and more preferably between 0.75 and 3 mm, is definable in each of the leather pieces.

To obtain the leather pieces according to the second size, a second reduction in the size of said pieces is totally or partially applied. Preferably, this reduction in the size of the leather pieces is carried out by a grinding, another, thereof. In this case, an impact grinder is used. Hence, the reduction, or grinding, is preferably applied only to the leather pieces with the maximum final length greater than 5 mm, and more preferably greater than 3 mm. Thus, a second uniformity in the size of the leather pieces is, therefore, obtained.

Performing the first grading and the second grading, in addition to the first and second reduction in the size of the leather pieces, provides total, or practically total, uniformity in the final size obtained. The greater the uniformity in the size of the leather pieces, the greater the uniformity in the quality of the final product obtained. In the event that only one of these gradings is applied, together with the corresponding reduction, the leather pieces can also be obtained according to an appropriate size, with the total application time of this method being reduced.

The method comprises dehydrating the leather pieces. For this purpose, the leather pieces are placed in a drying oven. In this sense, the leather pieces are exposed to hot air flows in the drying oven. The air is applied such that the leather pieces are disposed of leather with a temperature range of between 120°C and 285°C, more preferably between 155°C and 250°C, and even more preferably between 185°C and 220°C.

In order to ensure the temperature of the leather pieces in the dehydration thereof, an oven temperature control is performed so that the temperature of the leather pieces is maintained according to one of the aforementioned temperature ranges. Preferably, this control is performed according to different points of a containment part of said leather pieces.

The drying oven used is elongated, in other words, it has a longitudinal configuration for a progressive and continuous forward movement or displacement of the leather pieces from one end of the drying oven to the other end thereof. Likewise, in correspondence with the containment part of the leather pieces during their dehydration, said oven comprises fins with dual function.

On the one hand, the fins are disposed so that, together with a rotational displacement of the containment part with respect to an imaginary central longitudinal axis of the containment part of the leather pieces during dehydration, they displace the leather pieces along the longitudinal span of said containment part.

Furthermore, the fins are disposed so that, together with a rotational displacement of the containment part with respect to its imaginary central longitudinal axis, the leather pieces are moved about with one another for a uniform exposure of the latter to hot air flows.

In this sense, preferably, the oven is disposed according to a horizontal, or substantially horizontal, disposal according to the aforementioned imaginary central longitudinal axis of the containment part of the leather pieces during their dehydration.

The drying oven used is preferably made of stainless steel and/or galvanized steel. In other words, the oven is formed or manufactured in accordance with materials resistant to the oxidation generated by the moisture or steam produced by the drying of the leather pieces. Additionally, using the drying oven composed of stainless steel and/or galvanized steel provides adequate displacement of the leather pieces along it.

Likewise, in order to contribute to caring for the environment, the drying oven has filtering means, such as a particulate filter. In this way, the oven is configured to filter gases and fluids emitted in the drying of the leather pieces. Thus, it prevents hazardous waste or substances from polluting the environment. In this sense, the present method comprises obtaining the modified collagen product in a clean and sustainable manner, since the filter means implies that only a water vapour produced from drying is released into the environment.

This step of drying or dehydrating the leather pieces is performed so that the relative humidity thereof is reduced to a final value preferably between 0.5% and 8%, more preferably between 0.9% and 5.5%, and even more preferably between 1.5% and 4%, and yet even more preferably of 3%, or approximately 3%.

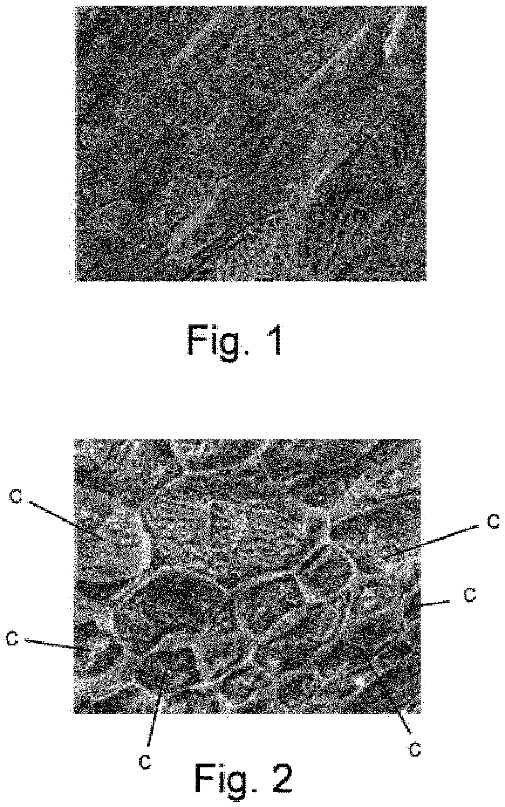

The leather pieces comprise collagen fibres. Figure 1 shows an enlarged view of one of the leather pieces prior to the application of the present method, such that a localization of the collagen fibres therein can be observed.

Collagen has physical properties including hydrophilicity, according to which it has a tendency to interact with water or to dissolve in it, or another polar substance. These molecules are capable of forming hydrogen bonds and are generally polarized. Polar and non-polar molecules are known as hydrophilic and hydrophobic, respectively.

Likewise, collagen is hydrophilous or hydrophilic, i.e., it is a substance that has affinity for water. In a solution or colloid, the hydrophilic molecules are in turn lipophobic; in other words, they cannot be mixed with lipids or fats.

In this sense, the disclosed step of drying or dehydration results in a denaturing of the collagen fibre of the leather pieces. This denaturing entails an alteration or modification of an internal structure typical of collagen.

Drying, or dehydration is carried out giving rise to this modification by breaking a collagen chain, changing its molecular structure so that it no longer has the physical property of hydrophilicity so that the collagen becomes, according to a physicochemical context, hydrophobic. In this way, the collagen is modified so that it is repelled by water, in addition to not being water-miscible. Thus, the product obtained is provided with a flotation capacity in water.

Likewise, this modification results in the collagen behaving as hollow cells (C) so that they can absorb, by capillarity, and store or retain all types of hydrocarbons and oils, both vegetable and synthetic. See Figure 2.

Thus, the collagen acquires properties that turn it into an oleophilic substance or product, in addition to hydrophobic. The modified collagen product obtained has an absorption capacity of up to 8 times its weight, according to ASTM F/726 analysis.

Additionally, the modified collagen product obtained has a specific gravity of approximately 0.8 or 0.9, an ash content of about 4% and inert matter of approximately 0.5%.

In this sense, the modified collagen absorbent product obtained is also flame-retardant; i.e., it does not generate flames, carbonizing at 280°C; and, biodegradable.

In order to provide an improved generation of the hollow cells (C), once the leather pieces have the relative humidity according to the final desired value, they are left to rest at ambient temperature for a progressive and continuous cooling.

Once the temperature of the leather pieces corresponds to the ambient temperature, preferably between 15°C and 25°C, the leather pieces, or already the modified collagen absorbent product from leather, is in a position to be packaged, stored and/or transported for its distribution.

The packaging is preferably performed in such a way that the modified collagen absorbent product from leather is placed in bags. Likewise, these bags comprise recycled paper.

According to one embodiment, and according to the steps described, a raw material acquisition, i.e., the collection of the leather pieces or shavings, is initially performed. Then, the raw material is placed outdoors during the corresponding time period so that the natural aeration of said raw material is carried out.

Likewise, preferably, after natural aeration fulfilling the conditions described above, the raw material is collected and undergoes the first size-grading described above. In this sense, the first mechanical sieving is performed so that the leather pieces are identified and selected according to the first size.

After the first grading, the raw material is placed in a large capacity metering accumulator or feeder, preferably of at least 1,000 kg (kilograms), more preferably of at least 1,500 kg, and even more preferably of at least 2,000 kg.

Preferably by means of a worm screw, the raw material is transferred from the accumulator or metering feeder to the drying oven. This transfer is performed in a dosed form. Once in the drying oven, the raw material is kept therein until the relative humidity reaches the final value according to what was described above.

Then, once said final value is reached, the raw material is transported to be ground so that the first reduction in size is carried out, preferably by means of a grinder. Preferably, this other grinder is referred to as the TT-160 grinder. This transport preferably takes place on one or more rubber belts.

After the first reduction, the resulting leather product is transported to be ground so that the second reduction in size is carried out, preferably by another grinder. Preferably, this other grinder is referred to as an impact grinder. This transport is preferably performed along one or more rubber belts in the "L" position.

After said second size reduction, said leather product is obtained according to the second size. In this sense, this ground leather product according to the desired maximum end length is displaced, preferably, by another worm screw to an additional rubber belt. Thus, the ground leather product is distributed to one, two or more silos so that it is stored waiting to be packed or packaged.

Subsequently, the ground leather product is transported, preferably via means comprising a worm screw, to the means for being packed or packaged. Once the ground leather product, which obviously corresponds to the modified collagen product, has been packed or packaged, it is ready to be distributed to intermediate or final consumers.

In view of the described embodiment, it is clear that the described steps can be performed according to different orders of succession. Likewise, some of them can even be eliminated, such as, for example, the first grading, the first reduction, the second grading and/or the second reduction, depending on the size of the leather pieces so that they are obtained according to the second size.

As such, the drying or dehydration step can take place before or after obtaining the leather pieces according to said second size.

## Claims

1. Method for obtaining a modified collagen absorbent product from leather, **characterized in that** it comprises the steps of:
- aerating flat leather pieces with a molecular structure during a time period between 15 and 45 days;
- collecting the leather pieces with an initial relative humidity value of 45% or less;
- grading the leather pieces by size;
- obtaining the leather pieces with a maximum final length of between 0.5-1.5 mm,
- placing the leather pieces in a drying oven;
- applying heat in the drying oven so that the leather pieces are exposed to a temperature of between 120ºC and 285°C during a time period,
- controlling the temperature applied during the time period so that the relative humidity is reduced to a final value between 0.5-5% in the leather pieces;
- reducing the temperature of leather pieces to an ambient temperature;
- obtaining the modified collagen absorbent product from leather.

2. Method according to claim 1, wherein the initial relative humidity value is equal to or less than 35%.

3. Method according to claim 1 or 2, wherein the grading of the pieces by sizes is performed by means of a mechanical sieving.

4. Method according to any one of claims 1 to 3, wherein the maximum final length obtained is between 0.75-3 mm.

5. Method according to any one of claims 1 to 4, wherein the drying oven is made of stainless steel and/or galvanized steel.

6. Method according to any one of claims 1 to 5, wherein the drying oven has fins for moving around and displacing the leather pieces along the same during the application of the temperature.

7. Method according to any one of claims 1 to 6, wherein the temperature in the drying oven is detected so that a regulation is carried out to keep the temperature of the leather pieces within a desired range.

8. Method according to any one of claims 1 to 7, wherein the drying oven has a particulate filter disposed to collect particles generated by the application of heat to the leather pieces.

9. Method according to any one of claims 1 to 8, wherein the time period of heat application is dependent on the applied temperature and the relative humidity of the leather pieces.

10. Method according to any one of claims 1 to 9, wherein the reduction in the temperature is performed by exposure to ambient temperature.

## Patentansprüche

1. Verfahren zur herstellung eines modifizierten kollagenabsorbierenden Produktes aus Leder, **dadurch gekennzeichnet, dass** die folgenden Schritte umfasst:
- Belüften von flachen Lederstücken mit einer Molekülstruktur über eine Zeitspanne von 15 bis 45 Tagen;
- Sammeln der Lederstücke mit einem Anfangswert der relativen Feuchtigkeit von 45% oder weniger;
- Einstufen der Lederstücke nach Größen;
- Erhalten der Lederstücke mit einer endgültigen Höchstlänge von 0,5-1,5 mm,
- Legen der Lederstücke in einen Trocknungsofen;
- Anwenden von Wärme in dem Trocknungsofen, sodass die Lederstücke einer Temperatur von 120°C bis 285°C über eine Zeitspanne ausgesetzt werden,
- Kontrollieren der über die Zeitspanne angewandte Temperatur, sodass die relative Feuchtigkeit in den Lederstücken auf einen endgültigen Wert von 0,5-5% reduziert wird;
- Reduzieren der Temperatur von Lederstücken auf eine Raumtemperatur;
- Erhalten des modifizierten kollagenabsorbierenden Produktes aus Leder.

2. Verfahren nach Anspruch 1, wobei der Anfangswert der relativen Feuchtigkeit gleich oder weniger als 35% beträgt.

3. Verfahren nach Anspruch 1 oder 2, wobei das Einstufen der Stücke nach Größen mittels eines mechanischen Siebens durchgeführt wird.

4. Verfahren nach einem der Ansprüche 1 bis 3, wobei die erhaltene endgültige Höchstlänge 0,75-3 mm beträgt.

5. Verfahren nach einem der Ansprüche 1 bis 4, wobei der Trocknungsofen aus Edelstahl und/oder verzinktem Stahl ausgebildet ist.

6. Verfahren nach einem der Ansprüche 1 bis 5, wobei der Trocknungsofen Flügel aufweist, um während der Temperaturanwendung die Lederstücke herum zu bewegen und entlang denselben zu versetzen.

7. Verfahren nach einem der Ansprüche 1 bis 6, wobei die Temperatur in dem Trocknungsofen erfasst wird, sodass eine Regelung durchgeführt wird, um die Temperatur der Lederstücke innerhalb eines gewünschten Bereiches zu halten.

8. Verfahren nach einem der Ansprüche 1 bis 7, wobei der Trocknungsofen einen Partikelfilter aufweist, der angeordnet ist, um die durch die Wärmeanwendung auf die Lederstücke generierten Partikeln zu sammeln.

9. Verfahren nach einem der Ansprüche 1 bis 8, wobei die Zeitspanne der Wärmeanwendung von der angewandten Temperatur und der relativen Feuchtigkeit der Lederstücke abhängig ist.

10. Verfahren nach einem der Ansprüche 1 bis 9, wobei die Temperaturreduzierung durch eine Aussetzung der Raumtemperatur durchgeführt wird.

## Revendications

1. Procédé pour obtenir un produit absorbant de collagène modifié à partir du cuir, **caractérisé en ce qu'**il comprend les étapes consistant à :
- aérer des pièces de cuir plates à structure moléculaire pendant un laps de temps compris entre 15 et 45 jours ;
- collecter les pièces de cuir avec une valeur initiale d'humidité relative de 45 % ou moins ;
- classer les pièces de cuir par taille ;
- obtenir les pièces de cuir avec une longueur finale maximale comprise entre 0,5 et 1,5 mm,
- placer les pièces de cuir dans une étuve de séchage ;
- appliquer de la chaleur dans l'étuve de séchage de sorte que les pièces de cuir soient exposées à une température comprise entre 120 °C et 285 °C pendant un laps de temps,
- contrôler la température appliquée pendant le laps de temps de sorte que l'humidité relative soit réduite à une valeur finale comprise entre 0,5 et 5 % dans les pièces de cuir ;
- réduire la température de pièces de cuir à une température ambiante ;
- obtenir le produit absorbant de collagène modifié à partir du cuir.

2. Procédé selon la revendication 1, dans lequel la valeur initiale d'humidité relative est égale ou inférieure à 35 %.

3. Procédé selon la revendication 1 ou 2, dans lequel le classement des pièces par tailles est effectué au moyen d'un tamisage mécanique.

4. Procédé selon l'une quelconque des revendications 1 à 3, dans lequel la longueur finale maximale obtenue est comprise entre 0,75 et 3 mm.

5. Procédé selon l'une quelconque des revendications 1 à 4, dans lequel l'étuve de séchage est réalisée en acier inoxydable et/ou en acier galvanisé.

6. Procédé selon l'une quelconque des revendications 1 à 5, dans lequel l'étuve de séchage a des ailettes permettant de bouger et de déplacer les pièces de cuir le long de celle-ci pendant l'application de la température.

7. Procédé selon l'une quelconque des revendications 1 à 6, dans lequel la température dans l'étuve de séchage est détectée de sorte qu'une régulation est effectuée pour maintenir la température des pièces de cuir dans une plage souhaitée.

8. Procédé selon l'une quelconque des revendications 1 à 7, dans lequel l'étuve de séchage a un filtre à particules disposé pour collecter des particules générées par l'application de chaleur aux pièces de cuir.

9. Procédé selon l'une quelconque des revendications 1 à 8, dans lequel le laps de temps d'application de chaleur dépend de la température appliquée et de l'humidité relative des pièces de cuir.

10. Procédé selon l'une quelconque des revendications 1 à 9, dans lequel la réduction de la température est réalisée par exposition à la température ambiante.
